# DEMANDE DE BREVET EUROPEEN

(11) **EP 0 633 018 A1**
(43) Date de publication de la demande: **11.01.1995**
(21) Numéro de dépôt: 94401270.7
(22) Date de dépôt: 08.06.1994
(51) Int. Cl.: A61K 7/48, A61K 7/06, C08L 83/06

(54) **Mélange silicone autoémulsifiable**

(30) Priorité: 09.07.1993 FR 9308502
(71) Demandeur: RHONE-POULENC CHIMIE, 92408 Courbevoie Cédex (FR)
(72) Inventeur: Willemin, Claudie, F-75018 Paris (FR)
(74) Mandataire: Seugnet, Jean Louis

(57) **Abrégé**

La présente invention concerne un mélange silicone autoémulsifiable caractérisé en ce qu'il comporte :
- 5 à 95 % en poids d' une huile silicone et,
- 95 à 5 % en poids d' un polydiméthylsiloxane polyoxyethyléné polyoxyalkylé de formule :

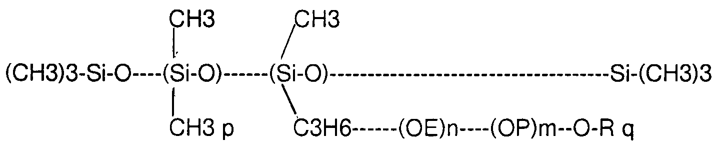

Utilisation des mélanges autoémulsifiables comme additifs dans les compositions cosmétiques en particulier dans les compositions capillaires et les compositions pour la peau.

## Description

La présente invention concerne un mélange autoémulsifiable utilisable notamment dans les compositions cosmétiques.

Les huiles silicones sont utilisées dans les compositions cosmétiques depuis de nombreuses années. Il en est de même pour les copolymères silicones polyoxyalkylénés. Par ailleurs il est déjà connu d' utiliser des associations huiles silicones/copolymères silicones polyoxyalkylénés dans des compositions cosmétiques.

Ainsi US 4 788 006 enseigne une telle association comportant en outre un tensioactif anionique et de la gomme xanthane. EP 155 806 décrit également dans les compositions cosmétiques une telle association comportant en outre une substance lipidique (alcool gras, esters gras) et un tensioactif cationique.

EP 152 953 décrit un concentré d' émulsionnant eau-dans-silicone destiné à être mélangé à de l'eau sous la forme d' une émulsion eau-dans-silicone comportant un copolymère silicone/polyol et une silicone cyclique.

Le but essentiel de la présente invention est de proposer un mélange à base de silicone sensiblement anhydre et qui soit directement autoémulsifiable par simple addition d'eau.

Un autre but de la présente invention est que l'émulsion immédiatement et aisémént obtenue soit stable et présente des particules huileuses dans l'eau monopopulées et et de granulométrie très ressérée.

Un autre but de la présente invention est l'utilisation d' un tel mélange ainsi que l'émulsion obtenue à partir d' un tel mélange, notamment dans les compositions cosmétiques et plus particulièrement dans les compositions pour cheveux et les compositions pour la peau.

Un autre but de la présente invention est de proposer un mélange de ce type qui permette d' obtenir après mélange avec de l'eau un mélange transparent optiquement clair.

Ces buts et d' autres sont atteints par la présente invention qui concerne en effet un mélange silicone autoémulsifiable caractérisé en ce qu'il comporte :
- 5 à 95 % , de préférence 30 à 70 en poids d' une huile silicone et,
- 95 à 5 % , de préférence 70 à 30 % en poids d'un copolyol

polydiméthylsiloxane polyoxyethyléné polyoxypropylé de formule : 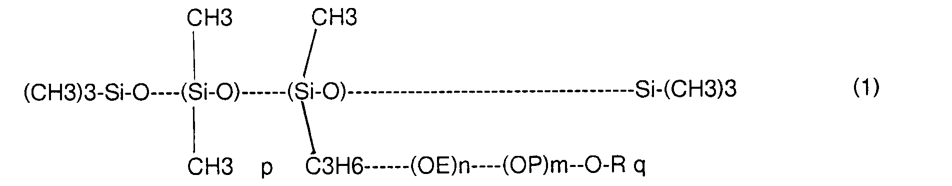
dans laquelle :
- p est un nombre entier compris entre 50 et 100, de préférence entre 65 et 85,
- q est un nombre entier choisi entre 5 et 10, de préférence entre 6 et 8,
- n est un nombre entier compris entre 15 et 30, de préférence entre 20 et 25,
- m est un nombre entier compris entre 15 et 40, de préférence entre 20 et 35,
- R est choisi parmi un atome d'hydrogène et un radical alkyle ou acyle linéaire ou ramifié ayant de 1 à 3 atomes de carbone,
- OE signifie O-CH2-CH2,
- OP signifie O-CH2-CH2-CH2,

Le copolymère de formule (1) peut présenter en outre de préférence au moins l'une des caractéristiques suivantes :
- la masse moléculaire en poids (Mw) est comprise entre 20 000 et 25 000,
- le rapport en poids C3H6------(OE)n----(OP)m--O-R/silicone est compris entre 2 et 3,5, de préférence entre 2, 5 et 3,0,
- le rapport OE/OP est compris entre 0,5 et 1,2, de préférence entre 0,7 et 1,0,
- R est un atome d' hydrogène ou le groupe acyle ( -CO- CH3).

On préfère plus particulièrement utiliser selon l'invention les copolymères répondant à la formule (1) ci-dessus et commercialisés par la société Rhône-Poulenc sous la dénomination commerciale SILBIONE 70646 et par la Société Dow Corning sous la dénomination commerciale DC 190.

L' huile silicone peut être choisie parmi les huiles silicones totalement insoluble ou au moins partiellement soluble dans l'eau. De plus ces huiles peuvent être volatiles ou non volatiles, cycliques ou essentiellement linéaires.

L' huile silicone peut donc être notamment n' importe quelle huile silicone sensiblement linéaire ayant une viscosité dynamique à 25 °C comprise entre 0,65 mPa.s et 2 000 000 mPa.s. Cette viscosité dynamique à 25 °C des polymères silicones est mesurée à l'aide d'un viscosimètre Brookfield selon la norme AFNOR NFT 76 102 de février 1992.

Cette huile silicone peut être un polydiorganosiloxane dont les radicaux organiques identiques ou différents sont des radicaux hydrocarbonés quelconques, et dans ce cas l'huile silicone est insoluble dans l'eau; cependant pour des raisons de disponibilité industrielle ces radicaux sont plus particulièrement choisis parmi les radicaux méthyle, éthyle, propyle, vinyle et phényle. Le mélange autoémulsifiable selon l'invention est plus particulièrement intéressant pour mettre aisément et rapidement en dispersion aqueuse une huile silicone visqueuse, c' est à dire une huile dont la viscosité est comprise entre 300 000 et 2 000 000, plus particulièrement entre 400 000 et 1 000 000 mPa.s.

L' huile peut comporter en outre dans la chaîne polymère ou en bout de chaine, un radical choisi parmi un groupe polaire relié à l'atome de silicium par une liaison Si-C ou Si-O-C et un radical silanol. Ce groupe polaire est de préférence un groupe aminé mais il peut être également choisi parmi un groupe amides, sulfonates, carboxyliques, phosphates ainsi que leurs sels. La présence de ces groupes polaires peuvent rendre l'huile silicone directement au moins partiellement soluble dans l'eau.

L'huile silicone peut être constituée en totalité ou en partie par une huile silicone cyclique qui est une cyclopolydiorganosiloxane dont le nombre de motifs diorganosiloxanes dans le cycle est compris entre 3 et 6 et dont les groupes organiques ont la même définition que celle indiquée plus haut pour les huiles linéaires.

Selon un mode préféré de mise en oeuvre de l'invention, tout ou partie de l'huile silicone, dans le mélange autoémulsifiable , peut être remplacé par un ester d' acide gras saturé ou insaturé parmi lesquels on peut citer le palmitate d' isopropyle, I' oléate de décyle, le laurate, le cocoate ou le caprate caprylate d'éthyl-2 héxyle. De même, jusqu'à 30 % en poids des huiles silicones ou du mélange huiles silicones/esters d' acides gras peuvent être remplacés par des huiles minérales ou végétales. Parmi les huiles végétales on recommande d'utiliser celles du type triglycérides telles que l'huile d'amande douce ,et l'huile de pépins de raisins ou une huile de type monoester telle que l'huile de jojoba.

Le mélange autoémulsifiable peut être préparé par simple mélange de l'huile silicone et du polydiméthylsiloxane polyoxyethyléné polyoxypropylé dans un ordre quelconque.

L' émulsion est ensuite préparée par simple addition d' eau en agitant de préférence. On recommande d'ajouter de 0,1 à 15, de préférence de 0,3 à 6 parties d'eau pour 1 partie de mélange silicone. Le mélange se fait avantageusement à température ambiante, dans un mélangeur muni d' une pale cadre raclante. L'émulsification se fait immédiatement et spontanément dès l'addition d' eau et on obtient des particules d' huile dans l'eau dont la granulométrie est généralement comprise entre 0,05 et 4 µm selon essentiellement le mode de mélangeage utilisé. En outre ces particules sont monopopulées et de granulométrie très ressérée. L' émulsion obtenue est stable au stockage et convient parfaitement à la préparation de compositions cosmétiques.

Un tel résultat aussi avantageux ne peut être obtenu qu' avec un copolyol répondant à la formule (1) ci-dessus, comme par exemple avec le copolyol Silbione 70 646 commercialisé par Rhône-Poulenc ou par le copolyol DC 190 commercialisé par Dow Corning. Par contre ce résultat surprenant n' est pas obtenu par exemple avec les copolyols 71 636 de Rhône-Poulenc et les copolyols Alkasil NE 58-50 et Alkasil NE 73-70 du commerce qui ne répondent pas à la formule (1) et qui conduisent à des dispersions grossières polydispersés c'est-à-dire dont la granulométrie n'est pas resserrée. Par granulométie insuffisamment resserrée on entend de préférence selon l' invention une émulsion dont au moins 20% des particules présentent une granulométrie inférieure ou supérieure à 7 à 10 fois de la valeur moyenne.

Par ailleurs selon un des modes de réalisation préféré de l'invention, on réalise un mélange autoémulsifié translucide et même transparent pouvant être en outre optiquement clair en adaptant les quantités d' eau de copolyol et d' huile afin de régler de façon adéquate l'indice de réfraction moyen du mélange. En outre l'utilisation d' une huile silicone au moins partiellement hydrosoluble facilite l'obtention d'un mélange autoémulsifié transparent optiquement clair qui est beaucoup recherché en cosmétique.

Pour préparer une composition cosmétique ou un mélange précurseur de mélange cosmétique, on peut ajouter à l'émulsion ou dispersion aqueuse préparée par simple mélange comme on l'a vu ci-dessus, au moins un tensioactif choisi parmi un tensioactif anionique, cationique, amphotère, zwitterionique, non ioniques et leurs mélanges possibles. Dans les compositions cosmétiques de lavage la teneur en tensioactif est généralement comprise entre 3 et 50 % en poids.

Comme tensioactif anionique on peut citer plus particulièrement : les sels alcalins, les sels d'ammonium, les sels d'amines ou les sels d'aminoalcools des composés suivants :
- les alkylsulfates, alkyléthersu Ifates, alkylamidesulfates et éthersulfates, alcanolamidesulfates, alkylarylpolyéthersulfates, monoglycéridesulfates,
- les alkylsulfonates, alkylamidesulfonates, alkylarylsulfonates, oléinesulfonates, parrafinesulfonates,
- les alkylsulfosuccinates, alkyléthersulfosuccinates, alkylamidesulfosuccinates,
- les alkylsulfosuccinamates,
- les alkylsulfoacétates, alkylisothionate et alkylaurate,
- les acylsarcosinates, acylpolypeptidates, acylamidopolypeptidates, le radical alckyle ou acyle de ces composés étant constitué par une chaîne carboné comportant 6 à 20 atomes de carbone.

Parmi les agents tensio-actifs anioniques, on peut également citer :
- les sels d'acides gras tels que les acides oléique, ricinoléique, palmitique, stéarique, les acides d'huile de coprah ou d'huile de coprah hydrogénée.
- des acyl lactylates dont le radical acyle comporte 8 à 20 atomes de carbone.

Parmi les agens tensio actifs non ioniques, on peut citer en particulier: les alcools, les alkylphénols et acides gras polyoxyéthylénés, polypropoxylés ou polyglycérolés à chaîne grasse comportant 6 à 20 atomes de carbone, le nombre de groupements d'oxyde d'éthylène ou d'oxyde de propylène étant compris enbtre 4 et 40 et le nombre de groupements glycérol étant compris entre 2 et 30.

On peut également citer les copolymères d'oxydes d'éthylène et de propylène, des condensats d'oxydes d'éthylène et de propylène sur des alcools gras, des amides gras polyéthoxylés (de préférence de 2 à 30 moles d'oxyde d'éthylène), des éthanolamides, des esters d'acides gras de glycol, des esters d'acides gras du sorbitan oxyéthylénés (de préférence de 2 à 30 moles d'oxyde d'éthylène) ou non, des esters d'acides gras du saccharose , des esters d'acides gras de polyéthylène glycol, des esters d'acides gras de dérivés du glucose, des amines grasses polyéthoxylées (de préférence de 2 à 30 moles d'oxyde d' éthylène), des oxydes d'amines, tels que les oxydes d'alkyamine.

Parmi les tensio-actifs non ioniques utilisables, on recommande les alkylspolyglucosides, les sucroglycérides éthoxylées et les alkylglucamides.

Parmi les agents tensio-actifs amphotères et zwittérioniques qui peuvent être utilisés, on peut citer les dérivés d'amines secondaires ou tertiaires aliphatiques, dans lesquels le radical aliphatique est une chaîne linéaire ou ramifiée comportant 6 à 18 atomes de carbone et qui contient au moins un groupe anionique hydrosoluble carboxylique, sulfonate, sulfate, phosphate ou phosphonate.

Parmi ces composés, on peut citer plus particulièrement les produits vendus sous la dénomination MIRANOL, décrits dans les brevets US-A 2 528 378 et 2 781 354. On peut également citer les alkylbétaïnes, les sulfobétaïnes, les amidobétaïnes ou les amidosulfobétaïnes.

Les alkylbétaïnes sont choisies de préférence parmi les alkyl (C10 - C 20) bétaïnes.

Conformément à la présente invention, on utilise de préférence en association avec le mélange autoémulsifiable des mélanges de tensio-actifs et en particulier des mélanges constitués de tensio-actifs anioniques ou des mélanges constitués des tensio-actifs anioniques associés à des agents tensio-actifs amphotères, zwittérioniques ou non-ioniques.

Lorsque les tensio-actifs amphotères ou zwittérioniques sont utilisés en mélange avec les tensio-actifs anioniques, ils représentent jusqu'à 60 %, et de préférence de 10 à 40 % en poids du total de la quantité d'agents tensio-actifs présents dans la composition.

Lorsque les tensio-actifs non-ioniques sont utilisés en mélange avec des tensio-actifs anioniques, ils représentent jusqu'à 90 %, et de préférence de 10 à 60 % du poids total de la quantité d'agents tensio-actifs présents dans la composition.

Les compositions cosmétiques ou leur précurseur peuvent également contenir des agents stabilisants qui peuvent être des hydrocolloides tels que la gomme xanthane, la gomme guar, la gomme de caroube, des galactomanames, des scléroglucanes et des alginates à une teneur de 0,05 à 3, de préférence de 0,1 à 2 % en poids par rapport au poids total de la composition cosmétique.

Elles peuvent contenir également des agents régulateurs de viscosité tels que des électrolytes et des dérivés de la cellulose et dérivés des polyéthylène glycol.

Les compositions cosmétiques peuvent également contenir différents adjuvants habituels tels que des parfums, des conservateurs, des séquestrants, des stabilisateurs de mousse, des agents propulseurs, des colorants, des agents acidifiants, des alcalanisants.

Les compositions peuvent se présenter sous forme de liquides plus ou moins épais, de gels,et de mousses aérosols.

Dans tout ce qui suit et ce qui précède , sauf indications contraires, les parties et pourcentages sont en poids et les viscosités sont mesurées à 25 °C.

Les exemples suivants illustrent l'invention sans en limiter la portée.

### Exemple 1:

### Préparation d' un mélange autoémulsifiable :

Dans un mélangeur muni d' une pale raclante, on mélange à température ambiante 80 parties d'huile silicone 70 047 V 500 000 commercialisé par Rhône Poulenc qui est une huile silicone polydiméthyle siloxane de viscosité 500 000 mPa.s et le copolyol 70 646 commercialisé par Rhône Poulenc qet répondant à la formule (1) ci-dessus avec p = 75, q = 7, n = 22, m = 24 et R = H. On obtient un concentré visqueux facilement émulsifiable par simple addition d' eau.

### Préparation d'un shampoing doux ayant la composition suivante :

| | |
|---|---|
| Sodium Laureth Sulfate ® | 36,0 % poids, |
| Disodium Cocoamphodiacetate MIRANOL C2M CONC NP ® | 4,0 % poids |
| Lauramide de diéthylène (DEA) | 2,0 % poids |
| Glycol distéarate | 2,0 % poids |
| Distéarate de PEG 6000 | 0,5 % poids |
| SILBIONE 70047 V 500000 ® huile polydiméthyl-siloxane de viscosité 500000 mPa.s | 1,0 % poids |
| Copolyol - SILBIONE 70646 formule 1 avec p = 75 , q = 7 ; n = 22 ; m = 24 et R = H | 0,25% poids |
| RHODICARE ® (gomme xanthane) | 0,35 % poids |
| JAGUAR HP 60 ® (gomme Guar) | 0,65 % poids |
| GERMABEN II ® (conservateur) | 0,5 % poids |
| Parfum | 0,25 % poids |
| Polysorbate 20 - ALKAMULS T 20 | 0,25 % poids |
| Eau distillée | q .s.p. 100 |

On disperse le RHODlCARE S ® et le JAGUAR HP 60 ® dans l'eau avec une pale défloculeuse. On hydrate le mélange sous agitation puis on ajoute à ce mélange sous agitation le lauryléther sulfate de sodium et le Miranol C2 M CONC NP ®. On maintient l'agitation jusqu'à l'homogénéisation.

On chauffe à 75°C et le distéarate de PEG 6000 (distéarate de polyéthylène glycol de Mw = 6000) est ajouté sous agitation modérée à l'aide d'une pale cadre ainsi que le glycol . Après fusion et dissolution, on maintient l'agitation jusqu'à retour à température ambiante et on ajoute le lauramide DEA (DEA = diéthylène amine). On ajoute alors le mélange autoémulsifiable préparé auparavant huile silicone/copolyol, le GERMABEN II ®, le parfum et l'ALKAMUS T20.

On règle le pH entre 6 et 7 par addition d'acide citrique.

On obtient ainsi un shampooing doux facilitant le démêlage et apportant de la brillance aux cheveux.

Ce shampoing se présente sous la forme d'un liquide visqueux, opaque, présentant une viscosité de 4000 mPa.s (Brookfield, aiguille N° 4, 10 t/mn).

Les particules huileuses de ce shampooing sont en émulsion stable monopopulée présentant une granulométrie resserrée voisin de 1 µm.

### Exemple 2 :

### Préparation d'un shampooing 2 en 1 :

On répète le mode opératoire de l'exemple 1 pour préparer la base lavante ayant la composition suivante et sans ajouter le mélange autoémulsifiable :

| | |
|---|---|
| RHODICARE S® (gomme xanthane) | 0,5 % poids |
| JAGUAR HP 60® (gomme guar) | 0,5 % poids |
| Lauryl éther sulfate de sodium | 36,0 % poids |
| Lauramide de DEA | 2,0 % poids |
| Glycol distéarate | 2,0 % poids |
| Distéarate de PEG 6000 | 1,5 % poids |
| Eau | 54,75 % poids |
| Conservateur GERMABEN II | 0,5 % poids |
| Parfum + ALKAMULS T/20 | 1,0 % poids |

On incorpore à cette base lavante 1,25 % en poids d'un mélange autoémulsifiable Silbione 70047V500000 et Silbione 70646 préparé au préalable suivant un rapport pondéral 80/20 par simple mélange à température ambiante dans un mélangeur munie d'une pale raclante suivant le mode opératoire décrit à l'exemple 1.

On obtient un shampooing dont les particules huileuses sont en émulsion stable monopopulées présentant une granulométrie resserrée voisine de 1 µm.

### Exemple 3:

### Préparation de mélanges autoémulsifiables conduisant à des émulsions transparente :

On répète le mode opératoire de l'exemple 1 concernant la préparation de mélanges autoémulsifiables en utilisant les huiles suivantes :

Huile H1 : Silbione 70041 V0,65 qui est de l'hexaméthyldisiloxae de viscosité 0,65 m.Pa.s.

Huile H2 : Silbione 7047 V20 à Silbione 70047 V500 000 qui sont des huiles sensiblement linéaires polydiméthylsiloxane de viscosité allant de 20 m.Pa.s à 500 000 m.Pa.s.

Huile Silicone volatile H3 : Silbione 71631 qui est un mélange SILBIONE 70045 V500000 dans une huile cyclique.

Huile silicone volatile H4 : Silbione 70633 V30 portent des groupes méthyle et phényle de viscosité 30 m.Pa.s.

On prépare des émulsions stables monopopulées, de granulométrie resserrée en suivant par exemple les compositions pondérales indiquées dans le tableau 1 ci-après :

**TABLEAU 1**

| EMULSIONS | HUILES SILICONES | POLYOL 70646 | EAU |
|---|---|---|---|
| E1 | H1 - 40 % | 20 % | 40 % |
| E2 | H2 - 45 % | 30 % | 25 % |
| E3 | H3 - 50 % | 25 % | 25 % |
| E4 | H4 - 18 % | 12 % | 70 % |

### Exemple 4:

### Préparation de gels solaires :

On prépare un mélange M1 de 5 % d'huile silicone H2 (Silbione 70047V500000 dans de l'huile silicone H3.

A partir de ce mélange M, on prépare, en suivant le mode opératoire de l'exemple 1, un mélange autoémulsifiable en mélangeant 18 parties de M1 à 40 parties de Silbione 70646.

On prépare 3 gels coulables translucides ayant les compositions indiquées dans le tableau 2 ci-après.

**TABLEAU 2**

| | GEL 1 | GEL 2 | GEL 3 |
|---|---|---|---|
| M1 | 18 | 28 | 38 |
| PARSAL MCX® (Filtre solaire) | 2 | 2 | 2 |
| Silbione 70646 | 40 | 35 | 30 |
| Eau | 40 | 35 | 30 |

## Revendications

1. Mélange silicone autoémulsifiable caractérisé en ce qu'il comporte :
- 5 à 95 % , de préférence 30 à 70 en poids d'une huile silicone et,
- 95 à 5 %, de préférence 70 à 30 en poids d'un copolyol polydiméthylsiloxane polyoxyethyléné polyoxypropylé de formule : 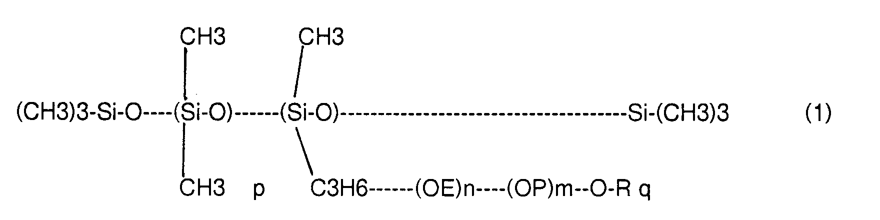 dans laquelle :
- p est un nombre entier compris entre 50 et 100, de préférence entre 65 et 85,
- q est un nombre entier compris entr 5 et 1 Ode préférence entre 6 et 8,
- n est un nombre entier compris entre 15 et 30, de préférence entre 20 et 25,
- m est un nombre entier compris entre 15 et 40, de préférence entre 20 et 35,
- R est choisi parmi un atome d'hydrogène et un radical alkyle ou acyle linéaire ou ramifié ayant de 1 à 3 atomes de carbone,
- OE signifie O-CH2-CH2,
- OP signifie O-CH2-CH2-CH2.

2. Mélange silicone autoémulsifiable selon la revendication 1, caractérisé en ce qu' il comporte en outre au moins l'une des caractéristiques suivantes :
le rapport en poids C3H6------(OE)n----(OP)m--O-R/silicone est compris entre 2 et 3,5, de préférence entre 2, 5 et 3,0,
le rapport OE/OP est compris entre 0,5 et 1,2, de préférence entre 0,7 et 1,0,
R est un atome d' hydrogène ou le radical acétyle.

3. Mélange silicone autoémulsifiable selon la revendication 1 ou 2, caractérisé en ce que l'huile silicone est une huile silicone cyclique ou sensiblement linéaire ayant une viscosité comprise entre 0,65 mPa.s et 2 000 000 mPa.s.

4. Mélange selon l'une quelconque des revendications 1 à 3, caractérisé en ce que l'huile peut comporter en outre dans la chaîne polymère ou en bout de chaine, un radical choisi parmi un groupe polaire relié à l'atome de silicium par une liaison Si-C ou Si-O-C et un radical silanol.

5. Mélange silicone autoémulsifiable selon l'une quelconque des revendications 1 à 4, caractérisé en ce que tout ou partie de l'huile silicone est remplacé par un ester d' acide gras saturé ou insaturé.

6. Mélange silicone autoémulsifiable selon l'une quelconque des revendicatons 1 à 5, caractérisé en ce que jusqu' à 30 % en poids des huiles silicones ou du mélange huiles silicones/esters d' acides gras est remplacé par des huiles minérales ou végétales.

7. Utilisation d' un mélange silicone autoémulsifiable tel que défini à l'une quelconque des revendications 1 à 6 pour la préparation de compositions cosmétiques.

8. Emulsions stables éventuellement transparentes de particules d' huile monopopulées et de granulométrie très ressérée, caractérisée en ce qu' elle est obtenue par simple mélange d' une quantité adaptée d' eau et de mélange silicone autoémulsifiable tel que défini à l'une quelconque des revendications 1 à 6.

9. Compositions cosmétiques aqueuses comportant une émulsion telle que définie à la revendication 8.
